# EUROPÄISCHE PATENTSCHRIFT

(11) **EP 1 421 906 B1**
(45) Veröffentlichungstag und Bekanntmachung des Hinweises auf die Patenterteilung: **11.01.2006**
(21) Anmeldenummer: 02025930.5
(22) Anmeldetag: 20.11.2002
(51) Int. Cl.: A61B 10/00

(54) **Sammelurin-Behältnis**
Urine collecting container
Récipient collecteur pour urine

(43) Veröffentlichungstag der Anmeldung: 26.05.2004
(73) Patentinhaber: Sarstedt AG & Co., 51588 Nümbrecht (DE)
(72) Erfinder: Sarstedt Walter, 51588 Nümbrecht (DE)
(74) Vertreter: Valentin, Ekkehard

(56) Entgegenhaltungen:
- AT-B- 340 040
- DE-A- 2 844 841
- NL-C- 1 014 931
- US-A- 4 873 193
- US-B1- 6 475 442

## Beschreibung

Die Erfindun betrifft ein Sammelurin-Behältnis, insbesondere eine Flasche, das einen Deckel und eine mit dem Deckel verschließbare, große Ein- und Auslaßöffnung besitzt, wie durch die US 6.475.442 B1 bekanntgeworden. Solche Behältnisse werden Patienten zur Verfügung gestellt, um über einen längeren Zeitraum deren abgegebenen Urin zur späteren Analytik für unterschiedliche diagnostische Zwecke zu sammeln.

Aus der DE 28 44 841 ist es bekannt, ein verschlossenes, ein medizinisches Präparat enthaltendes Versandröhrchen unter Klemmwirkung im Inneren eines Probenröhrchens, das durch einen Deckel verschließbar ist, zu halten.

Zur Sammlung des Urins sind einfache Kunststoffbehälter mit einem Fassungsvermögen zwischen 2 und 3 Liter üblich, die es ermöglichen, sogenannte 24-Stunden Sammelurine aufzunehmen. Dies erlaubt es, eine klinische Analytik aus einer Mischprobe aus einem über 24 Stunden gesammelten Urin durchzuführen. Die damit mögliche Langzeit-Sammlung verringert die Gefahr von Fehldiagnosen aufgrund von tageszeitlichen Schwankungen der Urinzusammensetzung. Dies ist besonders bedeutsam bei der Bestimmung der Konzentration von Katecholaminen und deren Abkömmlingen im 24 Stunden-Sammelurin. Die Bestimmung von Katecholaminen und ihrer Abbauprodukte im Urin ist nämlich für die Diagnose und Verlaufsbeurteilung von Tumoren des sympatho-adrenalen Systems, wie der Nebennierenrinde, von Bedeutung. Bei Vorliegen von Tumoren ist eine erhöhte Konzentration von Katecholaminen und deren Abkömmlingen im Urin zu finden, die bei der Synthese und Freisetzung allerdings tageszeitlichen Schwankungen unterliegen.

Bei der Bestimmung von Katecholaminen kommt noch hinzu, daß diese aufgrund ihres chemischen Aufbaus unter den üblichen Aufbewahrungs-Bedingungen, z.B. Raumtemperatur, Lichteinfluß und Zutritt von Luftsauerstoff, nicht stabil sind. Sie zerfallen langsam in andere Abbauprodukte. Die Analyse eines unter diesen Bedingungen gesammelten Urines würde zu falschen bzw. zu niedrigen Katecholaminwerten führen. Aus diesem Grunde sind die üblichen Sammelurin-Behältnisse, wie Flaschen oder Kanister, lichtundurchlässig und mit einem die Katecholamine stabilisierenden Reagenz versehen. Ein solcher Stabilisator ist aber beispielsweise bei Sammelurin zur Durchführung eines Nierensteintests nicht erforderlich, da nicht alle Sammelurine gesäuert werden.

Nachdem ein Patient die benötigte große Menge Sammelurin in den entsprechend großvolumigen Sammelbehälter abgelassen hat, wozu dieser mit einer großen Einfüll-Öffnung versehen ist, wird der Sammelbehälter von dem behandelnden Arzt einem Analysenlabor übermittelt, wo aus dem großen Sammelbehälter die für die Analytik benötigte, geringe Teilmenge entnommen wird. Beim Einsatz von üblichen Flaschen mit einem Volumen von 2 bis 3 Litern dient die große Einlaßöffnung gleichzeitig zum Ausgießen bzw. zur Entnahme der benötigten Sammelurin-Teilmenge. Selbst bei größter Sorgfalt ist beim Ausgießen einer kleinen Teilmenge in ein kleines Probengefäß ein Überschwappen nicht zu vermeiden. In jedem Fall ist es zudem nicht nur für den Patienten, sondern auch für den Arzt unumgänglich, zunächst den großvolumigen Sammelbehälter zu handhaben und aufwendig zu transportieren.

Wenn dann ein solcher Sammelbehälter außerdem mit einem stabilisierenden Reagenz befüllt werden muß, sind die betroffenen Personen durch die für die Ansäuerung in der allgemeinen Praxis üblichen Säuren wie Salzsäure, Schwefelsäure oder Eisessig außerordentlich gefährdet. Denn um einen Säuregrad in der vorhandenen großen Urinmenge von z.B. 3 Liter mit starker Pufferwirkung auch sicher zu erhalten, ist eine relativ große Menge einer der genannten Säuren notwendig, die dann häufig in der konzentriertesten im Handel üblichen Form eingesetzt werden. Alle bekannten Säuren werden in flüssiger Form entweder kurz vor Beginn der Urinsammlung vom Arzt oder unter Beachtung eines entsprechenden Gebrauchshinweises vom Patienten selbst in den Sammelbehälter gegeben. Je nach Handhabung und Sorgfalt des Anwenders sind Verätzungen nicht auszuschließen. Hierbei ergibt sich für den Patienten, wenn sich die Säure bereits von Beginn der Urinsammlung in dem Sammelbehälter befindet, aufgrund von Spritzem beim Urinlassen eine entsprechende Gefährdung.

Bei der allgemein üblichen Praxis läßt der Patient den Urin in den möglicherweise mit Säure additivierten, unhandlichen Unrinsammelbehälter der mit zunehmender Befüllung immer schwerer und damit immer unhandlicher wird. Eine solcherart durchgeführte Urinsammlung ist für die Patienten schwierig, insbesondere für weibliche Patienten.

Der Erfindung liegt daher die Aufgabe zugrunde, ein gattungsgemäßes Sammelurin-Behältnis zu schaffen, mit dem sich die beschriebenen Nachteile vermeiden und insbesondere die Handhabung vereinfachen sowie bei Verwendung eines Reagenz eine Personen-Gefährdung weitestgehend ausschließen lassen.

Diese Aufgabe wird erfindungsgemäß dadurch gelöst, daß ein für eine maximal zu erwartende Menge abgelassenen Urins bemessener, optional verformbar ausgebildeter Auffangbecher in die Öffnung des Sammelbehältnisses eingehängt und von dem Deckel beim Transport lagesicher gehalten ist. Hiermit läßt sich erreichen, daß dem Patienten ein gegenüber dem großvolumigen Sammelurin-Behältnis sehr viel kleinerer Behälter, nämlich der Auffangbecher mit etwa 400-500 ml Fassungsvolumen, zur temporären Nutzung sofort zur Verfügung steht. Denn im Anwendungsfall braucht der Patient nur den Deckel zu lösen und kann dann sogleich den mit einem radial nicht über den Öffnungsrand reichenden Kragen in das Sammelurin-Behältnis eingehängten Auffangbecher entnehmen und den Urin dann zunächst in diesen ablassen. Der Auffangbecher gestaltet das Auffangen des Urins sowohl für weibliche als auch für männliche Patienten sehr viel einfacher, ist deutlich leichter als der große Sammelurin-Behälter und dient anschließend sogleich als Umfüllgefäß zur Einfüllung der abgelassenen Urinmenge in das Sammelurin-Behältnis sowie später in ein Probengefäß. Das ohne größere Sorgfalt verschüttungsfreie Umfüllen des abgelassenen Urins wird durch eine elastisch verformbare Wandung des Auffangbechers begünstigt, da aufgrund der elastischen Wandung eine noch gezieltere Ausgußmöglichkeit vorliegt.

Das erfindungsgemäße Sammelurin-Behältnis ermöglicht in vorteilhafter Weise die Kombination mit einem Zubehörmittel, welches den für eine maximal zu erwartende Menge abgelassenen Urins bemessenen, optional verformbaren Auffangbecher umfasst, der zur Verwendung als Transportbehälter zur Aufnahme eines Stabilisatorfläschchens und/oder eines kleinvolumigen Probengefäßes verschließbar ausgebildet ist. Es läßt sich damit ein Nachrüst- bzw. Aufrüst-Kit für das großvolumige Sammelurin-Behältnis erreichen. Dies z.B. dann, wenn die Sammelurin-Behältnisse nach einmaligem Gebrauch nicht entsorgt werden, z.B. in Krankenhäusem, wo die Flaschen gespült und dann emeut gebraucht werden. Aber auch dann, wenn ein Sammelurin gesäuert werden soll, da die kleine Menge der dafür benötigten Säure mit dem in dem Auffangbecher beiliegenden Stabilisator- bzw. Säurefläschchen bereits zur Verfügung steht. Der z.B. mit einer Folie oder mit einem übergestülpten Deckel verschlossene Auffangbecher kann darüberhinaus auch eine Gebrauchsanweisung oder dergleichen aufnehmen.

Außerdem ist nach dem Sammeln des Urins über den notwendigen Zeitraum eine von der jahrzehntelang üblichen Praxis abweichende Handhabung möglich. Denn es braucht nicht mehr der befüllte großvolumige Sammelbehälter vom Patienten zum Arzt und von dort zum Analysenlabor transportiert zu werden, sondern nur noch das kleinvolumige Probengefäß, vorzugsweise ein für die Zentrifugierung geeignetes Röhrchen, in das aus dem Sammelbehälter unter Zuhilfenahme des mit einer verformbaren Wandung ausgebildeten Auffangbechers zuvor lediglich die für die Analytik benötigte geringe Teilmenge umgefüllt worden ist. Der Versand an das Analysenlabor beschränkt sich auf den Transport des kleinvolumigen Probengefäßes.

Der Auffangbecher als Zubehörmittel für ein Sammelurin-Behältnis stellt somit nicht nur einen Transport- und Bevorratungsbehälter für bei der Sammelurin-Gewinnung benötigte Mittel dar, wie Stabilisator, Probengefäß, Gebrauchshinweis,sondern läßt sich abgesehen von der erheblichen Erleichterung beim Urinablassen des Patienten als universelles Umfüllgefäß nutzen. Dies einerseits zum Befüllen des großvolumigen Sammelurin-Behältnisses und andererseits zur Umfüllung einer aus dem großvolumigen Sammelurin-Behältnis entnommenen Teilmenge in das kleine Probengefäß. ,

Weitere Einzelheiten und Merkmale der Erfindung ergeben sich aus den Patentansprüchen und der nachfolgenden Beschreibung von in den Zeichnungen dargestellten Ausführungsbeispielen der Erfindung. Es zeigen:
- Figur 1: in einem Teillängsschnitt einen in ein Sammelurin-Behältnis eingehängten Auffangbecher;
- Figur 2: im Längsschnitt einen Auffangbecher mit darin bevorratetem Probengefäß einschließlich Säurefläschchen; und
- Figur 3: eine der Figur 2 entsprechende Darstellung mit demgegenüber anderer Einlagerung der Zubehörmittel.

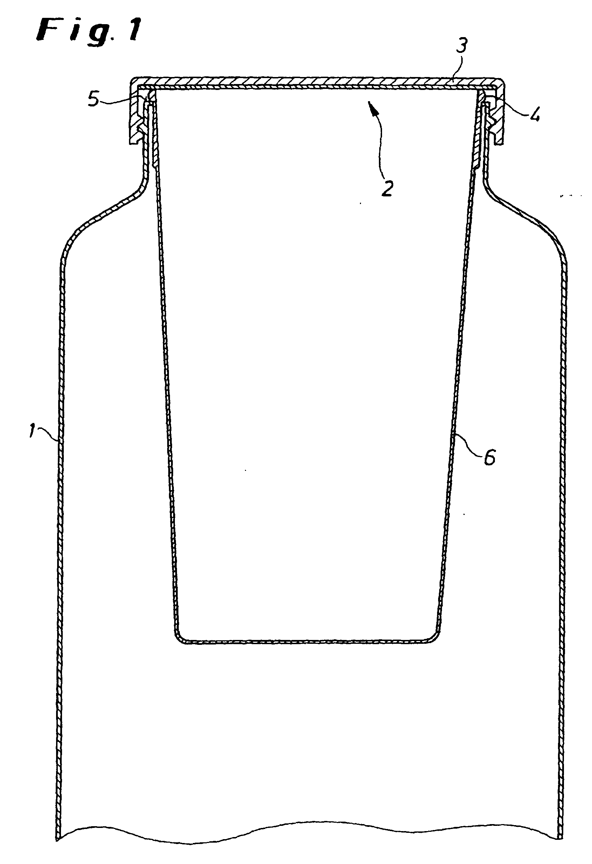

Die Figur 1 zeigt eine Teilansicht eines großvolumigen, hier als Flasche 1 zur Aufnahme von Sammelurin ausgeführten Sammelurin-Behältnisses. Die Flasche 1 besitzt eine große Ein- und Auslaßöffnung 2, die durch einen im Ausführungsbeispiel aufgeschraubten Deckel 3 dicht verschlossen ist. Ein in die Flasche 1 eingehängter, sich mit einem Umfangskragen 4 auf dem Öffnungsrand 5 abstützender Auffangbecher 6 wird von dem Deckel 3 lagesicher, in der Flasche 1 gehalten. Im Gegensatz zu dem Fassungsvolumen der Flasche 1 von 2000 bis 3000 ml besitzt der Auffangbecher 6 nämlich lediglich ein Fassungsvermögen von etwa 400 bis 500 ml.

Der einem Patienten mit der Flasche 1 sogleich zur Verfügung stehende Auffangbecher 6 ermöglicht es, daß der Patient den Urin nicht wie bisher üblich in den unhandlichen, großen Urinsammel-Behälter - Flasche 1 oder Kanister - abläßt, der mit zunehmender Befüllung immer schwerer und damit noch unhandlicher wird, sondern zunächst in den Auffangbecher 6. Aus diesem wird der Urin dann in die Flasche 1 gegossen. Durch die indirekte Befüllung der Flasche 1 mittels des als Umfüllgefäß dienenden Auffangbechers 6 wird außerdem die Gefahr der Verätzung empfindlicher Körperregionen aufgrund von Spritzern beim Urinlassen sicher ausgeschlossen.

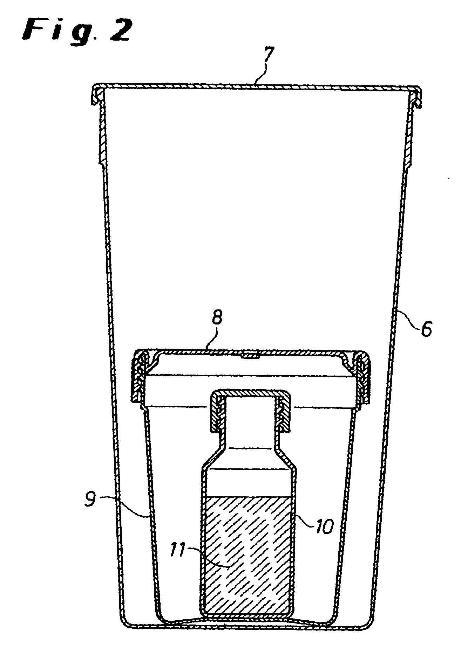

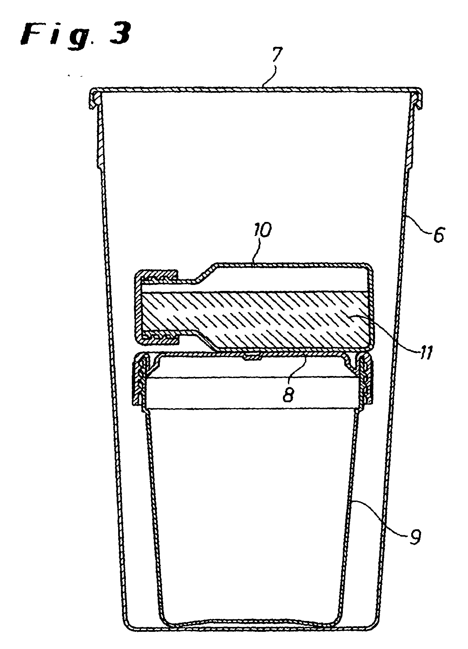

In den Figuren 2 und 3 sind in verschiedener Anordnung, ansonsten aber übereinstimmende Zubehörmittel für ein Sammelurin-Behältnis, wie insbesondere die vorbeschriebene Flasche 1, dargestellt. In jedem Fall ist ein zuvor beschriebener Auffangbecher 6 Basiselement des gezeigten Zubehör-Sets bzw.-Kits. Da der Auffangbecher 6 hier auch als Transportbehälter dient, ist seine Öffnung durch eine Folie oder einen aufgestülpten Deckel 7 verschlossen. In den Auffangbecher 6 eingelagert sind im Ausführungsbeispiel ein mit einem Deckel 8 verschießbares Probengefäß 9 und ein dicht verschlossenes Fläschchen 10 mit einem Stabilisator, insbesondere wie gezeigt eine Säure 11. Nach Figur 2 wird das Fläschchen 10 eingelagert in dem Probengefäß 9 und nach Figur 3 außerhalb des Probengefäßes 9 liegend in dem Auffangbecher 6 bevorratet.

Wenn eine Stabilisierung des Urins gewünscht wird, bietet es sich an, den Stabilisator bzw. die Säure 11 nach der erstmaligen Befüllung der Flasche 1, d.h. nachdem der Urin zuvor von dem Patienten in den kleinvolumigen, somit leichteren und zur besseren Handhabbarkeit optional radial verformbaren Auffangbecher 6 abgelassen und danach in die Flasche 1 umgefüllt wurde, zuzugeben. Hierdurch wird der unangenehme Nebeneffekt der Vorlage einer starken Säure, wie Rauchentwicklung und Geruchsbelästigung, vermieden. Außerdem wird die Gefahr der Verätzung durch die sofortige Verdünnung des Stabilisators entscheidend reduziert.

Durch den entweder von vornherein einen integrierten Bestandteil der Sammelurin-Flasche 1 (vgl. Figur 1) darstellenden oder nach den Figuren 2 und 3 mit weiterem Zubehör beigestelltem Auffangbecher 6 ergibt sich in jedem Fall für den Patienten eine sehr viel leichtere und - bei der Zugabe eines Stabilisators - ungefährlichere Handhabung mit universeller Umfüllmöglichkeit.

## Patentansprüche

1. Sammelurin-Behältnis, insbesondere Flasche (1), das einem Deckel (3) und eine mit dem Deckel (3) verschließbare, große Ein- und Auslassöffnung (2) besitzt,
**dadurch gekennzeiehnet,**
daß ein für eine maximal zu erwartende Menge abgelassenen Urins bemessener, optional verformbar ausgebildeter Auffangbecher (6) in die Öffnung (2) eingehängt und von dem Deckel (3) lagesicher gehalten ist.

2. Sammelurin-Behältnis nach Anspruch 1 in Kombination mit einem Zubehörmittel, welches den Auffangbecher (6) umfasst wobei dieser, zur Verwendung als Transportbehälter zur Aufnahme eines Stabilisatorgefäßes (10) und/oder eines kleinvolumigen Probengefäßes (9) verschließbar ausgebildet ist.

## Claims

1. A 24-hour urine container, especially a bottle (1), which comprises a cover (3) and a large inlet and outlet opening (2) which can be closed with said cover (3), **characterised in that**
a collecting beaker (6) embodied as optionally deformable and dimensioned for the maximum predicted quantity of discharged urine is suspended in the opening (2) and is held securely in place by the cover (3)

2. The 24-hour urine container according to claim 1 combined with an accessory means which comprises the collecting beaker (6), wherein said beaker is constructed as closable for use as a transport container for receiving a stabiliser vessel (10) and/or a small-volume sample vessel (9).

## Revendications

1. Récipient collecteur d'urine, notamment flacon (1) qui comporte un couvercle (3) et une grande ouverture d'entrée et de sortie (2) pouvant être fermée au moyen du couvercle (3),
**caractérisé en ce que**
un gobelet récupérateur (6) dimensionné pour une quantité maximale à attendre d'urine émise et réalisé de façon à être déformable en option est suspendu dans l'ouverture (2) et maintenu par le couvercle (3) en position sûre.

2. Récipient collecteur d'urine selon la revendication 1, en combinaison avec un accessoire qui entoure le gobelet récupérateur (6), celui-ci étant conçu avec possibilité de fermeture pour être utilisé comme récipient de transport de manière à recevoir un vase stabilisateur (10) et/ou un vase d'échantillon de petit volume (9).
